# EUROPEAN PATENT APPLICATION

(11) **EP 2 711 022 A2**
(43) Date of publication of application: **26.03.2014**
(21) Application number: 12785658.1
(22) Date of filing: 15.05.2012
(51) Int. Cl.: A61K 39/39, A61P 33/00

(54) **POLYMER MICROSPHERES AS ADJUVANTS IN THE PRODUCTION OF VACCINES AGAINST SCUTICOCILIATE FISH PARASITES**

(30) Priority: 19.05.2011 ES 201130806 P
(71) Applicant: Universidade de Santiago de Compostela, 15782 Santiago de Compostela (ES)
(72) Inventor: LEIRO VIDAL, José Manuel, E-15782 Santiago de Compostela (ES); LUZARDO ÁLVAREZ, Asteria, E-15782 Santiago de Compostela (ES); LAMAS FERNÁNDEZ, Jesús, E-15782 Santiago de Compostela (ES); LEÓN RODRÍGUEZ, Laura, E-15782 Santiago de Compostela (ES); BLANCO MÉNDEZ, José, E-15782 Santiago de Compostela (ES)
(74) Representative: Lorca Melton, Miguel
(86) International application number: PCT/ES2012/070345
(87) International publication number: WO 2012/156564

(57) **Abstract**

The invention relates to the use of microspheres as adjuvants in the production of a vaccine against scuticociliatosis. The invention provides a vaccine containing spherical chitosan microspheres and a copolymer of methyl vinyl ether and maleic anhydride, that act as an adjuvant, the microspheres being covered with an antigen of a membrane of a pathogenic turbot parasite, the scutiociliate *Philasterides dicentrarchi* (synonym of *Miamiensis avidus*). The vaccine can induce powerful immune responses and an increased protection in the turbot against scuticociliatosis, when it is administered parenterally, improving the protection induced by vaccines using oleaginous adjuvants containing metabolisable oils, but without generating unwanted side effects in the fish.

## Description

### FIELD OF THE ART

The present invention falls within the field of vaccine development for pathogens that affect species produced by aquaculture. More specifically, the invention describes the use of microspheres as adjuvants in the production of a vaccine against the scuticociliatosis.

The invention provides a vaccine containing chitosan microspheres and a copolymer of methyl vinyl ether and maleic anhydride, which act as adjuvant, the microspheres being coated with a membrane antigen of a pathogenic parasite of turbot, the scuticociliated *Philasterides dicentrarchi* (synonymous of *Miamiensis avidus*). The vaccine induces protection in fish, for example turbot, against scuticociliatosis and advantageously overcomes drawbacks in the immunization of other vaccines which use oily components as adjuvants.

### PRIOR ART

The development of new vaccines against emerging diseases of fish and improvement of conventional vaccine formulations has acquired great importance in recent years, especially due to the great impact caused in the economic and social fields of aquaculture (Sommerset, I. et al., 2005. Expert Review of Vaccines 4(1):89-101).

Conventional vaccine preparations typically include an antigen. The main drawbacks found are, on the one hand, the large amount of antigen required for immunization, and on the other hand, that protection obtained is generally poor and short-lived.

Adjuvants are pharmacological or immunological agents capable of increasing the effect of other agents, such as drugs or vaccines. Adjuvants are virtually useless if administered alone, but can be used to make a much more effective vaccine. When administered with a vaccine, an adjuvant stimulates the immune system and increases response to the vaccine. Therefore, the development of new adjuvant formulations to improve the immunogenicity of protective antigens and that enhance the immune response of fish is currently gaining a lot of interest.

Many pharmaceutical compositions of vaccines applied to fish include adjuvants to increase antigenic potency, also to improve the stability of the antigen or the formulation. In such cases, the available vaccine compositions often incorporate "depot effect" adjuvants by which adjuvants such as aluminum salts or water in oil emulsions, keep the antigen trapped at the administration site, allowing a prolonged immune stimulation. Such formulations, although improving the immune response of fish, carry some side effects when injected intraperitoneally such as: local inflammation, weight loss, or granulomas and abscesses formation, and thus, due to these drawbacks, use is often discouraged.

*Philasterides dicentrarchi* is an escuticociliated opportunistic parasite that causes a severe systemic infection in turbot, known as scuticociliatosis, invading organs and tissues (Iglesias R. et al. 2001 Diseases of Aquatic Organisms 46: 47-55). The parasite enters through skin injuries or through the gills (Parama A. et al. 2003 Aquaculture 217: 73-80) and invades different organs and tissues of the fish, causing serious injury which ultimately cause death. Scuticociliatosis is directly related to problems in water quality that favor the growth of the parasite (Emerging Diseases in Spanish marine aquaculture. 2007. Fish pathology service. Skretting. Autonomous University of Barcelona).

Immunoprophylaxis is presented as an alternative to chemotherapy, as it would allow the prevention of the infection since, at present, there are no effective drugs to treat it (Iglesias R. et al. 2008 Diseases of Aquatic Organisms 49: 417-424). Attempts have been made to obtain an effective vaccine against scuticociliatosis in different flatfish, but to the present date none of them has proved completely effective, especially due to the existence of different strains of ciliates and the inability to generate protection when immunized and infected with heterologous strains (Piazzon, C. et al. Fish & Shellfish Immunology 2008 25: 417-424). Some effective inactivated vaccine formulations exist, however, they use a large number of full ciliates (10⁶ ciliates/ml) inactivated with formalin and emulsified in Montanide type oily adjuvants (Lamas, J., et al. 2008. Aquaculture 278: 22-26) and whose safety has not been tested yet. These inactivated vaccines have disadvantages associated especially with the large amount of antigen used, the need to administer booster doses, and also, given the fact they are not subjected to any kind purification, contain all the biochemical components, which makes them in general more reactogenic than subunit vaccines, i.e., they cause more side effects (straying, L. 2002. Shots 3: 78-84).

For example, experiments carried out using Freund's incomplete adjuvant and 200 µg of *Philasterides dicentrarchi* immobilization antigen per individual, suggested that these antigens were not adequate to induce an immune response in flounder, demonstrating the difficulty in selecting suitable antigens for the development of scuticociliatosis vaccines (Lee, EH and Kim, KH 2008 Fish & Shellfish Immunology 24: 142e146). Turbot immunization trials using 400 µg of *Philasterides discentrarchi* antigen per individual with Freund's adjuvant did proved to be effective in protecting the fish from infection, but the toxicity of Freund's adjuvant makes it unsuitable for commercial application (Iglesias R. et al. 2003 Parasitology 126: 125-134). It is therefore desirable to obtain a vaccine to prevent fish scuticociliatosis which uses a less toxic adjuvant than those currently used, but that at the same time enhances the immune response, so that it is possible to incorporate the minimum amount of antigen, but sufficient to produce the immune response in fish.

In this regard, it is important that a subunit vaccine such as that proposed (i.e. without using full but parts of the ciliate), induces high levels of specific antibodies because, as shown recently, the effective control of ciliated parasite induced scuticociliatosis, is dependent mainly on the action of humoral components of the immune response of fish on the activation of the classical complement pathway, in which antibodies play a key role (Leiro J. et al, 2008 Parasite Immunology 30: 535-543). In fish, it has been shown that the peritoneal route of administration of vaccines generates the highest antibody response (Estevez J. et al. 1994. Aquaculture 123: 197-204).

It is known that P. dicentrarchi presents immunogenic antigens in its membrane that turbot is able to recognize and produce antibodies against them in response to a natural infection or tested under different immunization protocols (Piazzon C. et al 2008 Shelfish Fish and Immunology 25: 417-424).

The microparticles, as vaccine delivery systems, have the advantage of possessing a size similar to that of the pathogens which the immune system combat, and also are readily internalized by antigen presenting cells (O'Hagan DT, et al size. Methods 2006 40: 10-19). Furthermore, it has been shown that microparticles with encapsulated antigens exhibit a similar inmunogenecity to that of Freund's adjuvant (González Regueiro JR, et al immunogenicity. 2002. Immunology. Biology and pathology of the immune system. 3rd Edition. Panamérican medical Editorial, Chapter 7).

The preparation of microparticles with encapsulated antigens by methods or techniques conventionally used in the field of pharmaceutical technology sometimes cause the degradation of the active substance, mainly due to denaturation by the use of high temperatures, solvents, or high shear forces which are used in the preparation of micron-sized particles. It would therefore be desirable to provide microparticles capable of retaining the antigen without degrading it, for it to be recognized by the immune system.

The surface modification or functionalization of microparticles with molecules that can be recognized by macrophages would be an additional advantage to their capability as adjuvant. Thus, a vaccine would be obtained with adjuvants (microparticles) that would not generate the inconveniences produced by the oily adjuvants and, moreover, induce an effective protective response. This would allow far increased safety in immunoprophylaxis and a better control of scuticociliatosis in breeding flatfish, particularly turbot.

To this end, the present invention focuses on the preparation of a vaccine composed of microspheres obtained from biocompatible and biodegradable materials which carry on their surface covalently attached antigenic subunits from the plasma membrane of scuticociliated P. *dicentrarchi.* This pharmaceutical composition is parenterally administrable and is characterized by its special immunopotentiating capacity without generating adverse inflammatory reactions, providing adequate protection for fish against parasitic infectious diseases.

### DETAILED DESCRIPTION OF THE INVENTION

The present invention describes the preparation of polymeric microspheres, the composition of the polymeric microspheres, the pharmaceutical composition containing these microspheres, and the use of said composition in the preparation of a vaccine or a medicament for stimulating the immune response in fish.

The authors of the present invention have carried out an optimization process of the microsphere composition formed by two biodegradable and biocompatible polymers, so that when such polymers are in certain proportions, provide microspheres with stability so they are stable as dry powder (allowing the storage and preservation) and once reconstituted as aqueous suspension, maintaining all properties until administered. Moreover, the polymers forming part of the microspheres are biodegradable and biocompatible, that is, once administered, the microspheres of the invention are degraded in the body, leading to non-toxic metabolites that can be removed by normal metabolic pathways. Also, the microspheres are spherical and are of a particular size range, enabling their recognition by macrophages involved in the immune response. Moreover, the authors have demonstrated that the microspheres of the invention to which an antigen binds on the surface, are capable of increasing the immune response against the antigen bound to the microsphere, protecting the receptor against a specific disease and without adverse reactions for the host organism, particularly due to the low amount of antigen used.

In particular, the authors of the present invention have synthesized stable microspheres formed by biodegradable and biocompatible polymers (chitosan polymer and a copolymer of methyl vinyl ether and maleic anhydride) and which carry on their surface an antigen obtained from membrane components of the scuticociliated *Philasterides dicentrarchi,* wherein the antigen is covalently attached to the microspheres.

In a first aspect, the invention relates to microspheres, hereinafter "microspheres of the invention", comprising (i) a biodegradable polymer of chitosan or a derivative thereof, (ii) a biodegradable copolymer of methyl vinyl ether and maleic anhydride, or a derivative thereof, (iii) a crosslinking agent and (iv) one or more antigens.

In the present invention, the term "biodegradable polymer" refers to a polymer that can be degraded *in vivo* through the enzymatic route or not (a non-enzymatic pathway would be, for example, one were water penetrates into the microspheres, they are eroded and the remains are reabsorbed by the organism), and that as a result of degradation within the body, give rise to non toxic metabolites that can be removed by normal metabolic pathways.

In the present invention, the term "biocompatible polymer" refers to one that does not produce other kind of allergic reactions, immune reactions or adverse reactions when in contact with body tissues, i.e., that when placed in contact with a living organism, it is not harmful therefor.

In the present invention, the term "microsphere" refers to a spherical microparticle and having a matrix structure. Microparticle means particle with a diameter less than 1 mm, preferably less than 100 µm, preferably below 50 µm, and more preferably with a diameter between 1 and 10 µm. It is important to note that according to their internal structure and their morphology, the microparticles are classified into microcapsules and microspheres. The difference between both is that, while the microcapsules have a solid or liquid core coated with a polymeric membrane, in microspheres the system's structure is a matrix, and the shape is always spherical. Therefore, the present invention relates only to the particular type of microparticle called microsphere.

The microspheres may have an electric surface charge (measured by zeta potential), the magnitude of which can take positive or negative values depending on the proportion of the different components in the system. Both, the size and the zeta potential, of the microspheres are important parameters for the interaction with the surface of cells. In a particular embodiment of the invention, the microspheres have a positive charge that can vary between 30 mV and 50 mV, more preferably between 35 and 45 mV. The charge is particularly suitable to ensure the stability of the microspheres after parenteral administration. By covalently binding an antigen on the surface of the microsphere, the zeta potential thereof is increased, so that the increase in this parameter confirms the modification of the microsphere with the antigen, as demonstrated in the examples of the present invention. The zeta potential of the microspheres of the invention can be measured using standard procedures known to the skilled artisan, and which are described, for example, in the experimental part of the present specification.

Once synthesized, the microspheres of the invention are stable, i.e., maintain their stability in the form of lyophilized dry powder, and also during reconstitution and once reconstituted as aqueous suspension at room temperature, before being administered.

The first polymer that is part of the microsphere of the invention is a chitosan biodegradable polymer. Chitosan is a natural polysaccharide which is composed of units of beta-(1-4)-D-glucosamine (deacetylated unit) and N-acetyl-D-glucosamine (acetylated unit) arranged at random.

It is a nontoxic and biodegradable derivative of chitin. The polymer of chitosan used to prepare the microspheres of the present invention may have a molecular weight between 15 and 300 kDa, preferably between 25 and 200 kDa, more preferably between 50 and 100 kDa.

In the present invention, chitosan comprised in the microspheres may also be a derivative of chitosan. Included within the definition of chitosan derivative are the chitosan inorganic salts which can form the chitosan with acids, such as hydrochloric acid, acetic acid, lactic acid, glutamic acid, or citric acid. Such salts are water soluble, and they may be a way to incorporate chitosan into a pharmaceutical composition for use as a vaccine or for stimulating the immune response in an individual. In the examples of the present invention, chitosan hydrochloride is used, and is dispersed in a solution of acetic acid. Examples of chitosan derivatives include: O-acetylated, O-alkylated or O-sulfonated chitosans, carboxymethyl chitosan, chitosan having different degrees of acetylation, or cyclodextrin, galactose, mannose, phthalates, fatty acids or methylpyrrolidone derivatives, among others.

The second component part of the microspheres of the invention is a copolymer of methyl vinyl ether and maleic anhydride. An example of a commercial synthetic copolymer of methyl vinyl ether and maleic anhydride is Gantrez®AN, excipient widely used in the pharmaceutical and cosmetic industry. The ES2178961 B1 patent document describes other examples of copolymers of methyl vinyl ether and maleic anhydride commercially available.

The microspheres of the invention may be prepared with copolymers of methyl vinyl ether and maleic anhydride of various molecular weights, for example those sold under the name Gantrez®AN. The molecular weight of the copolymer of methyl vinyl ether and maleic anhydride used in the microspheres of the invention may be between 100 and 2000 kDa, more preferably between 100 and 300 kDa, and most preferably is 200 kDa. Derivatives of methyl vinyl ether and maleic anhydride may be Gantrez in its acid form (Gantrez S varieties).

The microspheres further comprise one or more antigens. Therefore, the microsphere may comprise a single antigen derived from a single organism, or several antigens which belong to different organisms, or different components of the same organism.

The term "antigen" refers to any substance capable of being recognized by the immune system of a subject and/or capable of inducing in a subject a humoral immune response or a cellular immune response leading to the activation of lymphocytes B and/or T when is introduced into a subject, for example, said term includes any immunogenic, native or recombinant product obtained from a higher organism or from a microorganism, for example, a bacterium, a virus, a parasite, a protozoa or a fungus which contains one or more antigenic determinants, for example, compounds excreted by these organisms, such as toxins, or structural components of these organisms, such integral components of the plasma membrane. Virtually any antigen can be used in the manufacture of microspheres of the invention coated with antigen. Preferably, the antigen which binds to the surface of the microspheres is an antigen from a parasite, even more preferably is an antigen from a parasite affecting cultivated in aquaculture fish, such as *Philasterides dicentrarchi, Miamiensis avidus, Uronema nigricans, Uronema marinum, Pseudocohnilembus persalinus, Ichthyophthirius multifiliis* or *Cryptocaryon irritans.;* more preferably is an antigen from at least one of the scuticociliated parasites Philasterides *dicentrarchi* or *Miamiensis avidus,* even more preferably, the antigen is a membrane component of the parasite; still more preferably, is a membrane antigen of at least one of the scuticociliated parasites *Philasterides dicentrarchi* or *Miamiensis avidus.*

In a preferred embodiment, the antigen(s) is(are) attached covalently to the outer surface of the microsphere.

Covalent bonding to bind the antigen to the microsphere can be established between the antigen and the remaining glutaraldehyde which exists on the surface of the microspheres. Furthermore, it can also be established between the antigen and the aldehyde groups of the copolymer of methyl vinyl ether and maleic anhydride. In the examples of the present invention, antigen binding to the surface of the microspheres is done by incorporating a solution of antigen over a microsphere suspension, keeping constant orbital agitation for 15 minutes at 22°C.

In a preferred embodiment of the invention, the antigen to be covalently bonded to the outer surface of the microsphere is added in a proportion of between 0.05 and 0.15% w/v. More preferably, the ratio of antigen is 0.1% w/v, meaning % w/v or weight/volume as the ratio between the amount of antigen in weight and the total volume of the suspension wherein the microspheres are suspended and antigen is dissolved, multiplied by 100. In the examples of the present invention, the antigen is incorporated into the microspheres by incubating 1 mg of antigen in a suspension of 10 mg of microspheres, the concentration of which is 10 mg/ml, i.e., the antigen concentration is 1 mg/ml or 0.1 % w/v in the volume of microspheres.

In another preferred embodiment, the microspheres have an amount of antigen covalently linked to its outer surface of between 2 and 7% w/w, preferably between 3 and 6% w/w, more preferably between 4 and 5% w/w , and even more preferably 4.5% w/w, meaning % w/w the ratio between the weight of the antigen and the weight of the microsphere, multiplied by 100.

In another preferred embodiment of the invention, the antigen covalently bonded to the outer surface of the microsphere is a component of a parasite. More preferably, it is a component of one of the parasites *Philasterides dicentrarchi, Miamiensis avidus, Uronema nigricans, Uronema marinum, Pseudocohnilembus persalinus, Ichthyophthirius multifiliis* or *Cryptocaryon irritans.* More preferably it is a component of at least one of the parasites *Philasterides dicentrarchi* or *Miamiensis avidus,* even more preferably, a component of the parasite membrane of at least one of the scuticociliated parasites *Philasterides dicentrarchi* or *Miamiensis avidus.*

*Philasterides dicentrarchi* is the causative agent of fish scuticociliatosis, and mainly affects turbot, flounder, sole, sea bass, wide-eyed flounder and hirami. In addition, several studies indicate that other scuticociliated such as *Miamiensis avidus* are phylogenetically close species to *Philasterides dicentrarchi,* having found up to 99% similarity between the two species in the sequence of the ribosomal RNA small subunit (Parama et al. 2006. Parasitology 132: 555-564).

The microspheres further comprise a crosslinking agent to improve the stability thereof, for example to improve its stability in an aqueous medium.

Illustrative non-limiting examples of crosslinking agents that can be used include compounds with aldehyde groups (such as glutaraldehyde or formaldehyde), sodium tripolyphosphate and other polyanions, genipin, glyoxal, ethylene glycol diglycidyl ether, and generally any molecule having functional groups capable of reacting with the functional groups present in the biodegradable polymers.

In a preferred embodiment, the crosslinking agent is glutaraldehyde, formaldehyde, sodium tripolyphosphate, genipin, glyoxal, ethylene glycol diglycidyl ether, preferably glutaraldehyde. Preferably, the microspheres are prepared by adding the crosslinking agent in a proportion of between 0.01 and 0.1 % w/v, more preferably 0.015 to 0.025%, and most preferably, in a proportion of 0.02% , the % w/v or weight/volume percentage understood as the ratio between the weight of the crosslinking agent and the total volume of the dispersion of the two polymers, multiplied by 100.

In the examples of the present invention, microspheres are prepared by dispersing, in the first place a certain amount of vinyl methyl ether and maleic anhydride copolymer in acetone, which is incorporated over a dispersion of chitosan hydrochloride in acetic acid, and to which a crosslinking agent which is added. Then an antigen solution is incorporated over a suspension of microspheres and the microspheres with bound antigen are collected by centrifugation.

In a preferred embodiment of the invention, the microspheres of the invention are prepared by adding the chitosan polymer in a proportion of between 0.12 and 0.5% w/v, preferably at a concentration of 0.25% w/v, and vinyl methyl ether and maleic anhydride copolymer in a proportion of between 0.15 and 0.5% w/v, preferably at a concentration of 0.24% w/v, % w/v or weight/volume percentage being understood as the ratio between the weight of the polymer or copolymer and the total final volume of the dispersion of the two polymers (100 ml in the examples of the present invention) multiplied by 100. These ranges of w/v percentages of the polymer and copolymer as well as the order in which the various components are added, the amount of crosslinker used, the crosslinking time, solvents and conditions of preparation (atomization temperature, flow rate of the polymer dispersion, and air pressure) are important so that the microspheres remain stable. Essays carried out by the authors of the present invention demonstrated that when the polymer and the copolymer bind in proportions outside these ranges, for example, result in less stable microspheres than those optimized within these ranges, thus the weight/volume percentages of each of them have to be taken into account to obtain suitable microspheres for storage as powder and subsequent reconstitution while maintaining all their properties until they are finally administered.

In a preferred embodiment of the invention, the microspheres have a diameter of between 1 to 10 µm, more preferably between 3.88 and 4.68 µm.

The spherical shape and size of the microspheres is an important characteristic for the manufacture of a pharmaceutical composition which is suitable for use as a vaccine or for stimulating the immune response in a subject, since this form of microsphere is more suitable for detection by macrophages, so that the power of the immune response produced in an individual is increased, and also the most suitable to be injected as part of a pharmaceutical composition.

In the present invention, the term "individual" or "subject" is understood as a member of a species of animal, preferably a vertebrate, more preferably a fish, more preferably a flatfish, and includes but is not limited to breaded marine fishes such as turbot, flounder, sole, sea bream, sea bass, Wide-eyed flounder and hirami.

In a preferred embodiment, the solid microspheres comprise:
i. Chitosan polymer, preferably of molecular weight between 50-100 kDa: in a proportion of between 25 and 75%, preferably between 35 and 60%, more preferably 47% by weight.
ii. Copolymer of methyl vinyl ether-maleic anhydride, preferably Gantrez AN119: in a proportion of between 25 and 75%, preferably between 35 and 60%, more preferably 45% by weight.
iii. Crosslinking agent, preferably glutaraldehyde: in a proportion of between 2.4 and 5%, preferably between 2.9 and 4.5%, more preferably 3.7% by weight.
iv. Antigen, preferably an antigen from a parasite affecting fish: in a proportion of between 2 and 7%, preferably between 2 and 6%, more preferably 4.5% by weight.

The weight percentage of each component is understood as the ratio between the weight of each component and the total weight of the solid microspheres multiplied by 100.

In the examples of the present invention, microspheres are obtained carrying an amount of antigen covalently bonded to the outer surface of the microsphere of 46 µg of antigen/mg of microparticle (4.6 wt% or 4.6% w/p) which are able to induce an effective immune response in turbot. Given that the dose used for vaccination is 5 mg of microspheres, 230 µg of antigen per fish was administered, which is a particularly low quantity of antigen compared to other immunization attempts conducted in the prior art for fish scuticociliatosis.

In another preferred embodiment of the invention, the microspheres are lyophilized. The microspheres of the invention can be lyophilized by conventional methods. From a pharmacological point of view, it is important to have lyophilized microspheres, as this improves its storage stability and long-term conservation and additionally reduces the volume of product to be handled. The microspheres of the invention may be lyophilized in the presence of a usual cryoprotectant agent such as glucose, sucrose, mannitol, trehalose, glycerol, lactose, sorbitol or polivinilpilorridona, among others, at a concentration comprised within a wide range, preferably between 0,1% to 20% by weight. The microspheres can also be lyophilized without cryoprotectant such as in the examples of the present invention.

The microspheres of the invention may be administered to the individual in the form of aqueous suspension or as part of more complex pharmaceutical compositions, for example in conjunction with a suitable vehicle for the administration route.

Therefore, in another aspect, the invention relates to a pharmaceutical composition, hereinafter "pharmaceutical composition of the invention" comprising microspheres of the invention together with a dispersion medium. These dispersion media include, for example, water, saline solutions, phosphate buffered saline (PBS) solutions, or any solution suitable for containing the microspheres for subsequent administration. In a preferred embodiment, said dispersion medium comprises a suspending vehicle or dispersing agent suitable and accepted for route parenteral administration.

Suitable dispersing media for parenteral administration are, for example, aqueous solution of carboxymethylcellulose or polysorbate. Other suitable dispersion media can also be oily solutions of lecithin, paraffin, natural oils or other media such as microemulsions (nanometric size droplet emulsions comprised by an aqueous phase, oil phase, surfactant and co-surfactant). After adding the dispersant, the dispersion of the microspheres in the suspension medium can be effected by vigorous shaking of the vial containing the preparation, thereby forming a homogeneous suspension for injection.

In a preferred embodiment, the dispersion medium comprises carboxymethylcellulose, polysorbate, lecithin, paraffin wax or a natural oil, preferably the dispersion medium comprises carboxymethylcellulose.

In a preferred embodiment, the pharmaceutical composition comprises between 0.1-2% w/v of microspheres, preferably between 0.5-1.5% w/v, more preferably between 0.75 and 1.25% w/v, and most preferably comprises 1% w/v of microspheres, w/v percentage being understood as the ratio between the weight of the microspheres and the total volume in which the same are resuspended (e.g. in a suitable dispersion medium for the administration route for which it is intended) before being administered, multiplied by 100.

The pharmaceutical composition of the invention may also comprise other adjuvants incorporated into the suspension in order to enhance the immunopotentiating activity, which can be soluble or not in biological fluids.

In another preferred embodiment, the pharmaceutical composition of the invention comprises:
i. Chitosan polymer, preferably of molecular weight between 50-100 kDa: 0.25-0.75% (w/v)
ii. Methyl vinyl ether-maleic anhydride copolymer, preferably Gantrez AN119: 0.25-0.75% (w/v)
iii. Crosslinking agent, preferably glutaraldehyde: 0.024 to 0.05% (w/v).
iv. Antigen, preferably an antigen from a parasite affecting fish: 0.02-0.07% (w/v).
v. dispersion medium, preferably a solution of carboxymethylcellulose.

In another aspect, the invention relates to the use of the pharmaceutical composition of the invention for the manufacture of a medicament for stimulating and/or inducing an immune response in a fish.

In another aspect, the invention relates to the use of the pharmaceutical composition of the invention for the manufacture of a vaccine for fish. In a preferred embodiment, the fish are selected from the group comprising flatfish and bass. Among flatfish, turbot (*Scophthalmidae* family, e.g. *Psetta maxima, Scophtalmus maximus, Scophtalmus aquosus*), flounder (*Pleuronectidae* family, e.g. *Platichthys flesus*), plaice (*Pleuronectes platessa*), dab (Zeugopterus *regius, Phrynorhombus norvegicus*) browny (*Zeugopterus punctatus*), brill (*Scophtalmus rhombus*), megrim (e.g. *Lepidorhombus boscii* or *Lepidorhombus whiffiagonis*), flounder (*Soleidae* family, e.g. Solea *vulgaris*), wide-eyed flounder (*Bothus podas*), scaldfish (*Arnoglossus laterna*) and olive flounder (*Paralichthys olivaceus*); bass is understood as the family of Serranids, e.g. *Dicentrarchus labrax,* preferably fish are flatfish, such as turbot, flounder, dab, plaice, browny, brill, megrim, sole, wide-eyed flounder, scaldfish and olive flounder, more preferably, the fish is turbot or flounder.

In the pharmaceutical composition of the invention, the microspheres act as an antigen's adjuvant. An adjuvant is any substance that when incorporated in a vaccine, accelerates, prolongs or enhances the immunogenic response against the same.

In a preferred embodiment, the pharmaceutical composition is in a form adapted for parenteral administration. One way to accommodate the pharmaceutical composition comprising the microspheres to parenteral administration, is to incorporate microspheres in a suitable dispersion for parenteral administration, such as a carboxymethylcellulose solution.

Parenteral administration is one that is made by injection or infusion. Examples of parenteral administration include subcutaneous, intravenous, intraarticular or intramuscular injection.

In a preferred embodiment, the pharmaceutical composition of the invention is administered in a therapeutically effective amount. The term "therapeutically effective amount" refers to an amount of substance sufficient to achieve the intended purpose. The dose of microspheres of the invention to be administered to an individual may vary over a wide range, for example between about 0.1 and 0.5, preferably between 0.1 and 0.3, more preferably is 0.2 g/kg of body weight.

In another aspect, the invention relates to the process for preparing microspheres of the invention, comprising the steps of:
i. Preparing a dispersion of a copolymer of methyl vinyl ether and maleic anhydride in an organic solvent
ii. Preparing a dispersion of a chitosan polymer in an aqueous solvent
iii. Incorporating the dispersion of step i) with the dispersion of step ii)
iv. Addition of a crosslinker
v. Obtaining microspheres by one of the procedures selected from the group comprising: atomization, emulsion/solvent evaporation or coacervation.
vi. Antigen binding to the outer surface of the microspheres.

In a preferred embodiment of the invention, in step v), the microspheres are obtained by atomization. Preferably, the atomization process takes place under the following conditions: atomization temperature: 72°C, feed rate: 3-4 ml/min and air pressure, 500 l/h.

In another preferred embodiment, in step i), the organic solvent is acetone, and methyl vinyl ether copolymer is incorporated at an initial concentration of between 0.15 and 0.5% w/v, preferably 0.24% w/v relative to the volume of dispersion of the two polymers.

In another preferred embodiment, in step ii), the solvent is an acetic acid solution, preferably at a concentration of 0.1 M, and the chitosan used is chitosan hydrochloride at an initial concentration of between 0,12 and 0.5% w/v, preferably 0.25% w/v relative to the volume of dispersion of the two polymers.

In another preferred embodiment, in step iii), the mixture is carried out with magnetic stirring.

In another preferred embodiment, in step iv), the crosslinking agent is glutaraldehyde at a ratio of between 0.01 and 0.1% w/v, preferably between 0.015 and 0.025% w/v, more preferably 0.02 % w/v relative to the total volume of the dispersion of the two polymers.

In another preferred embodiment, in step vi) the binding of the antigen to the outer surface of the microsphere is of covalent type. Preferably, the antigen binding in step vi) is performed by incorporating an antigen solution at a ratio of between 0.05-0.15% w/v over a suspension of microspheres. Preferably, the antigen binding in step vi) is performed by incorporating an antigen solution at 0.1% w/v over a suspension of microspheres obtained in step v), under stirring for a certain period of time at 22°C and then centrifugating so as to isolate the microspheres.

The surprising finding that the polymeric microspheres can induce immune responses in turbot, is essential for the invention since it provides a new strategy for the control of parasitic diseases in commercial hatcheries. Furthermore, these vaccine compositions comprising microspheres can be used with suitable biocompatible dispersion media for parental administration. Also, in order to enhance the immunopotentiating activity, these formulations of the invention can be used with other adjuvants incorporated in the suspension of microspheres, these capable of being soluble or insoluble in biological fluids, that is, formulations containing microspheres can be administered both in aqueous suspension as an oily suspension.

### BRIEF DESCRIPTION OF THE FIGURES

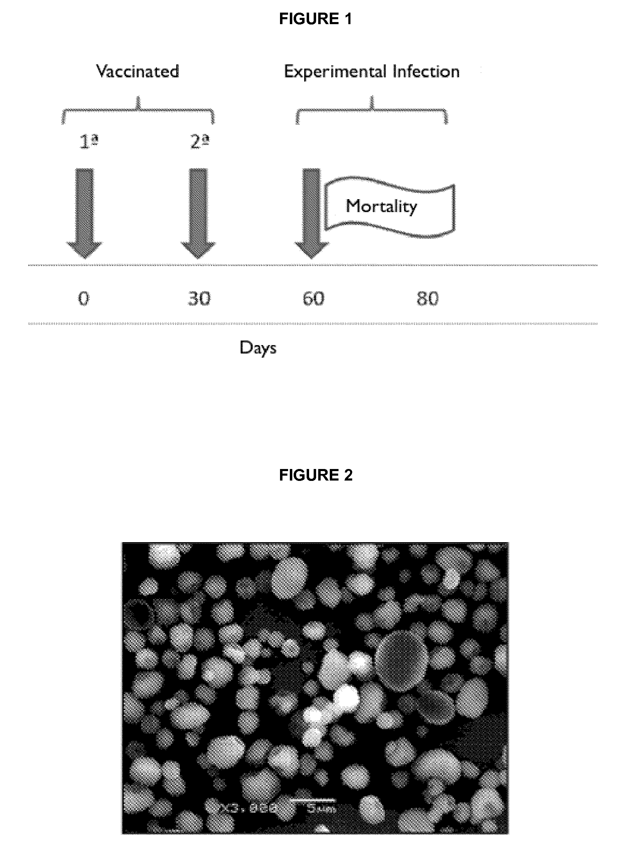

**Figure 1****:** experimental protocol of vaccination and determination of protection.
**Figure 2****:** Chitosan microspheres Seacure 210 HCl - Gantrez®AN119 (3000 X).

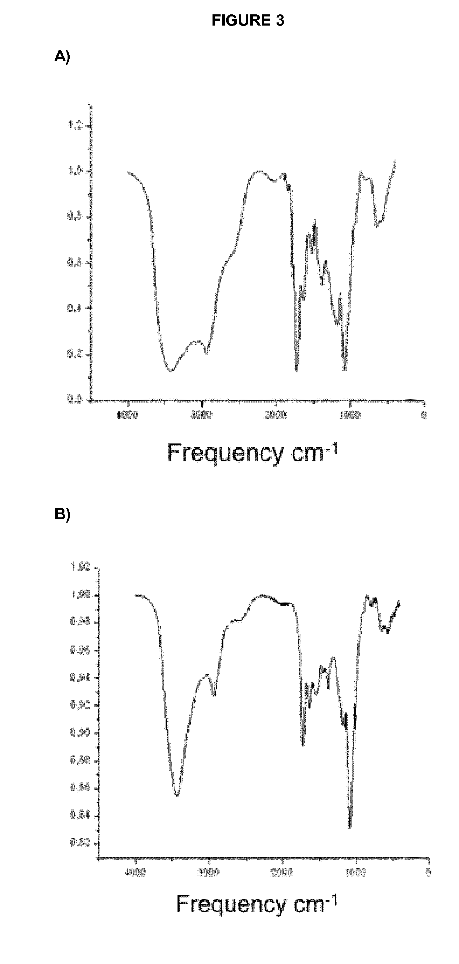

**Figure 3****:** Infrared spectrum of microspheres Chitosan Seacure HCl 210 and Gantrez®AN119 (a) and with the protein covalently bound to its surface (b).

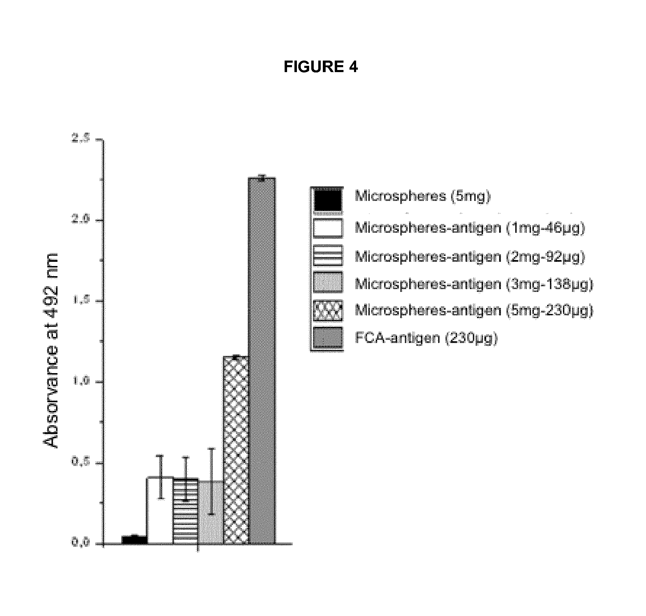

**Figure 4****:** Production of antibodies determined by ELISA. Mice were immunized intraperitoneally with a dose of 0.5 ml containing 1, 2, 3 and 5 mg of microspheres to which membrane antigen of *P. dicentrarchi* is covalently bound. The concentration of antigen bound to the microspheres follows the relationship of 46 µg of antigen/mg of microparticle. For the group that was immunized with Freund's complete adjuvant (FCA), each vaccine contains the highest concentration of antigen which is inoculated with the microspheres (230 µg). Bars indicate standard error of mean levels of antibodies (n = 5).

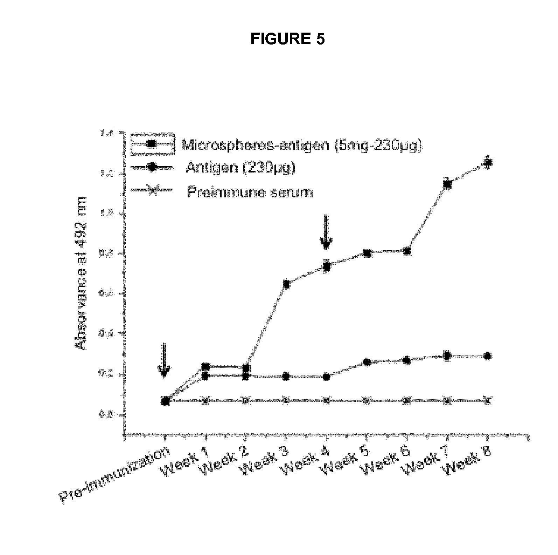

**Figure 5****.** Kinetics of antibody production in immunized turbot with 5 mg of microspheres containing 230 µg of antigen covalently bound to its surface, and immunized only with antigen. The antibody levels were quantified weekly using a double indirect ELISA essay, and each point represents the mean levels of antibodies from a pool of sera from 5 fish. Arrows indicate the days when immunizations were performed.

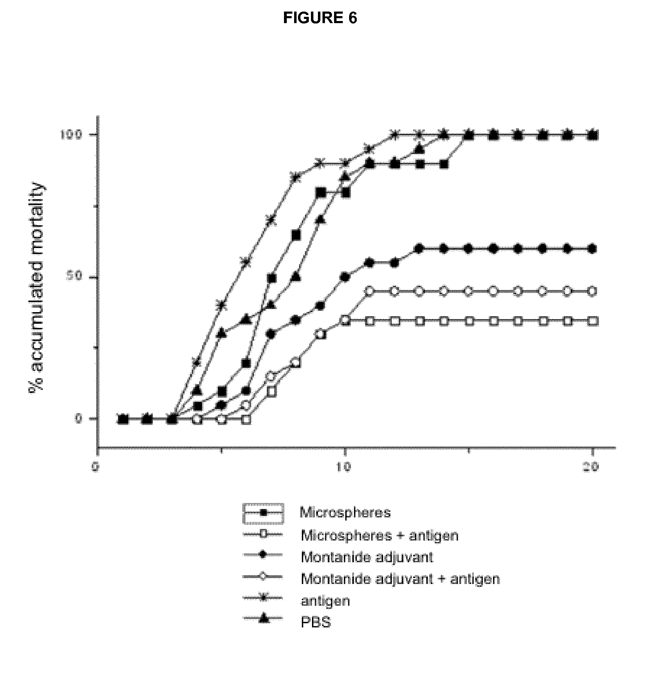

**Figure 6****:** Kinetics of mortality in fish experimentally infected with *P. dicentrarchi,* vaccinated on day 0 and 30 with different vaccine formulations including membrane antigen of *P. dicentrarchi* and as microspheres and oil formulations adjuvants (Montanide ISA 763a). Values are expressed as percentage of cumulative mortality for 20 days.

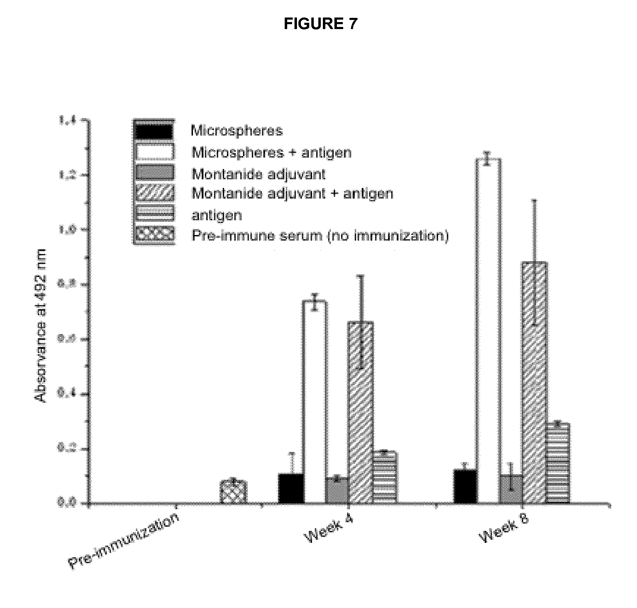

**Figure 7****:** Levels of antibodies produced by vaccinated turbots with various formulations containing membrane antigen (230 µg) of *P. dicentrarchi* covalently coupled to beads (5 mg/fish) or emulsified (1:1, v/v) in Montanide oil adjuvant. The determination of the antibodies was performed by an ELISA essay on sera collected on day 0 before immunizing fish (preimmune serum) and at 30 and 60 days postimmunization. Fish were individually bled and the sera obtained were pooled together. Results show the mean values ± standard deviation (n = 3).

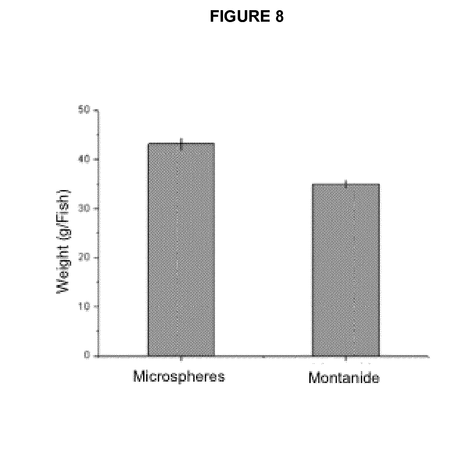

**Figure 8****.** Difference in the weights of the fish immunized with 5 mg of microspheres carrying membrane antigen *P. dicentrarchi* on its surface and those vaccinated with the same amount of antigen administered in an oil emulsion of Montanide. * Value of two-tailed P <0.0001, considered extremely significant when the two means are compared using the unpaired student t test (n = 20).

### EXAMPLES

The present invention is suitably illustrated by the following examples which are not intended to be limiting of its scope.

### MATERIALS AND METHODS

### Microsphere formation process

It is essential for the preparation of microspheres to select biopolymers with biodegradable properties, and thus of the microspheres. They are an example of this type of biopolymer chitosan and Gantrez in anhydride form. Chitosan hydrochloride and Gantrez AN 119 based microspheres are prepared by dispersing, first, 240 mg of Gantrez AN 119 in 50 ml of acetone, which is incorporated into a solution of 250 mg of Chitosan Seacure 210 HCl in 50 ml of 0.1 M acetic acid. 1 ml of 2% glutaraldehyde is used as crosslinking agent. The microsphere preparation can be carried out by various procedures such as atomization, emulsion/solvent evaporation or coacervation. The procedure for obtaining the microspheres takes place under the following atomization conditions: inlet air temperature (72°C), feed rate at 3-4 ml/min and air pressure at 500 l/h. Microspheres so prepared have a mean size of between 1 and 10 µm in diameter.

### Collection and purification of the antigen

The antigen is obtained as follows. Briefly, ciliates (*P. dicentrarchi,* strain I1) were washed 3 times with phosphate buffered saline solution (PBS, pH 7.4) buffer by centrifugation at 700 x g.

They were resuspended in PBS with 250 mM sucrose, and 3 mM phenylmethanesulfonyl fluoride (PMSF, a protease inhibitor) and were ultrasonically lysed in a Branson W-250 sonifier (Branson Ultrasonic Corporation, USA) on ice (8 sonication cycles of 10 pulses each using a 50% of duty cycle and output intensity four - between each cycle it was allowed to stand in ice for one minute). Then the pellet was centrifuged at 8000 x g for 5 minutes and resuspended in 3 ml of HEPES buffer (30 mM HEPES, 20 mM KCI, 5 mM MgSO4, 0.5 mM EDTA, pH 7.6) which was carefully applied on the top of a sucrose gradient with 3 different concentrations (in a centrifuge tube of 15 ml): Part A: 3 ml of 1.5 M (nuclei and ciliates) Part B: 3 ml of 1.0 M (membrane fraction) Part C: 3 ml of 0.5 M (cytoplasm and microsomes). This sucrose gradient was centrifuged at 6000 x g for 10 minutes very carefully withdrawing membrane antigen accumulated in Part B with a pipette. The antigen obtained was washed 2 times with PBS (11,000 x g for 10 minutes). Then, the antigen's integral membrane proteins obtained with Triton X-114 were extracted. For this, the pellet was incubated with 0.5% Triton X-114 for 1 hour at 0°C. Finally centrifuged at 10,000 x g for 10 minutes and stored at -20°C.

### Bonding procedure of the antigenic components to the microspheres

According to the invention, in a second step, the binding of the antigen to the surface of the microspheres is conducted as follows: The microspheres, based on Chitosan hydrochloride and Gantrez AN 119, are prepared by dispersing, in the first place, 240 mg of Gantrez AN 119 in 50 ml of acetone, which is incorporated into a solution of 250 mg of Chitosan Seacure 210 HCl in 50 ml of acetic acid 0.1 M. 1 ml of 2% glutaraldehyde was used as crosslinking agent. Next, the microspheres are obtained through some of the aforementioned techniques (atomization, emulsion/solvent evaporation or coacervation). Then a solution of 1 mg of antigen is incorporated over 10 mg of a suspension of microspheres and this suspension is kept under constant stirring at 500 rpm for 15 minutes. After this time, the microspheres were collected by centrifugation at 5000 g for 10 minutes and washed twice with PBS and a final time with water and then are lyophilized and stored at 4°C.

### Suspension in the dispersion medium

The microspheres carrying the antigen on their outer surface were incorporated to a dispersion medium suitable for parenteral administration. Suitable dispersing media for this type of administration are, for example, aqueous solutions of polysorbate or carboximentilcelulosa. Other suitable dispersion media which can also be used are oily solutions of lecithin, paraffin, natural oils or other media such as microemulsions (nanomeric size droplet size emulsions formed by an aqueous phase, an oily phase, surfactant and co-surfactant). In the examples of the present invention 1% carboxymethylcellulose was added as a dispersing agent. Once the dispersant had been added, the dispersion of microspheres in the suspension medium can be effected by vigorous shaking of the vial containing preparation, thereby forming a injectable homogeneous suspension.

### Vaccination protocol and obtaining immune serum

The administration of the microspheres with the antigen on their surface is performed by parenteral route, once resuspended in the dispersion medium. The administration was carried out following the protocol shown in Figure 1. Two separate vaccinations were made for 30 days each. Thus, each fish or mouse with a weight of 25 g, was vaccinated with a suspension of 5 mg of microspheres carrying 230 µg of membrane antigen coupled to its surface and in a total volume of 500 µl of buffer phosphate buffered saline solution (PBS) containing 1% carboxymethylcellulose (dispersing agent). Fish were also vaccinated with vaccines containing oily adjuvants (Seppic, Paris, France Montanide ISA 763 A) or mice with Freund's adjuvant and the same concentration of antigen (230 µg). Controls immunized only with the adjuvants and with the buffer containing the dispersing agent were included in all cases. To specifically determine the protective capacity of the vaccine, fish were experimentally infected on day 60 after immunization, each fish with 500 µl of medium L-15 containing 7.5 x 10⁵ ciliates. The cumulative mortality of vaccinated and control fish was monitored over 20 days post-infection.

Blood samples were collected from the caudal vein of the fish the same day of immunization and experimental infection (0, 30 and 60 days, see Figure 1). To determine the kinetics of antibody, fish were bled weekly for 8 weeks. These blood samples were kept two hours at room temperature and, in order to obtain the serum, and centrifuged at 3000 x g for 10 minutes. The obtained serum was diluted 1:1 in glycerol and stored at -20°C until later analysis.

To determine the effect of vaccination on the fish growth, all turbot vaccinated with microspheres and oil adjuvant were weighed on day 0 and day 60.

### Determination of the specific immune response

The determination of specific antibodies against the antigens of *P. dicentrarchi* present in the serum of vaccinated turbot was performed using an ELISA inmunoenzime essay. A 1 µg solution of antigen in 100 µl of coupling buffer 0.1 M NaHCO₃ /0.5 M NaCl was incorporated into each well of a 96-well microplate, and was allowed to incubate overnight at 4°C. Then it was washed with distilled water, and the wells were blocked with TBS-Tween 0.2% and 5% skim milk for 2 hours at 37°C in a humid chamber. Subsequently, dilutions of the immune serum of turbot diluted in a solution of TBS-Tween 0.2% and 1% skim milk were added and incubated for 2 hours at 37°C. After incubation the wells were washed 5 times with TBS buffer, and an anti-Ig turbot (UR3) monoclonal antibody at a 1:1000 concentration was added in a solution of TBS-Tween 0.2% and 1% skim milk, and incubated for 2 hours at 37°C in a humid chamber. Once incubated, the wells were washed 5 times with TBS, and then an antibody of rabbit anti-Ig of mouse conjugated with peroxidase diluted 1:2000 in 0.2% TBS-Tween buffer was added and incubated for 2 hours at 37°C. After this time, the wells were washed five times with TBS. The reaction was developed by adding to each well a solution of orthophenylenediamine (ODP) prepared in citrate buffer pH 5.1, containing 0.001% of H₂O₂ and incubated for 20 minutes. After this time, the reaction was stopped by adding 25 µl/well of H₂SO₄ 3N, and the test was read on a spectrophotometer plate reader at a wavelength of 492 nm.

### Example 1. Characterization of microspheres with antigen

### Physicochemical properties of the microspheres with membrane antigen of P. dicentrarchi Size, morphology, z potential. Quantification of acid groups

The size of the microspheres was determined through laser light scattering using a Mastersizer Micro (Malvern Instruments, UK). The microspheres were resuspended in distilled water and measurements were made at room temperature with constant agitation. The size distribution was made considering the volume occupied by the particles and was taken as average particle size of size value possessed by 50% of them. The size values that were above 90% and below 10% were used to estimate the extent of the size distribution.

The measurements of the surface charge of the microspheres were made with the equipment Zeta Plus Potential Analyzer (Brookhaven Instruments Corporation, New York, USA). The zeta potential was determined by ten measurements of each sample and as a result a mean and standard deviation were obtained. Likewise, each measurement was carried out in triplicate.

The shape and surface of the microspheres were examined by sweep electron microscopy photographs (LEO 435 VP, LEO Electron Microscopy Ltd., Cambridge, UK). To do this, dry samples of each formulation were placed on a double-sided adhesive sheet attached to a support and each sample was coated with gold using a Biorad E5000 metallizer. It is well established that the particle surface is smooth, non-porous, and that are mostly spherical (Figure 2).

IR studies were conducted in order to confirm the covalent bonding between the acid groups of the surface of the microspheres and the protein. The samples were prepared in KBr discs and transmittance between 400 and 4000 cm-1 was measured on a Bruker, model IFS-66 V.

Determination of the bound antigen was determined by the bicinchoninic acid method. For this purpose 10 mg of microspheres were dissolved in 3 ml of 0.1 M acetic acid overnight. After that, the sample was centrifuged at 20,000 x g for 20 minutes and the supernatant was spectrophotometrically measured at 562 nm by the bicinchoninic acid colorimetric method for proteins.

Table 1 shows the physicochemical characteristics of the microspheres obtained. The microspheres are characterized by size and zeta potential since these are important parameters for the interaction with the surface of cells. The microspheres have an average size of 4.28±0.4 µm, which gives a capacity to be internalized by antigen presenting cells, and a zeta potential that is increased when the microspheres carry on their surface antigen from 38.11±0.22 mV to 42.98±0.87 mV, confirming the modification of the microspheres.

**Table 1. Physicochemical properties of the microspheres**

| | Average | Standard deviation |
|---|---|---|
| Size | 4,28 µm | 0,4 |
| Zeta potential of microspheres of chitosan and Gantrez | 38,11 mV | 0,22 |
| Zeta potential of microspheres of chitosan and Gantrez with antigen on its surface | 42,98 mV | 0,87 |
| Protein amount bound to the surface | 45,7 µg/mg of microparticle | 0,9 |

Figure 3 shows the infrared spectrum of the microspheres of Chitosan Seacure 210 HCl and Gantrez®AN119 with protein on the surface. Comparing the spectra, one can observe a decrease in the absorption band at 3400 cm⁻¹ corresponding to the aldehyde groups of glutaraldehyde containing microspheres. When the antigen is bound to the surface (Fig. 3b), the peak disappears because the amino groups of the antigen react with the aldehyde groups, resulting in amide formation.

Decrease of the absorbance peak at 1700 cm⁻¹ is due to binding of the protein with the free carbonyl groups of Gantrez®AN119. The variation in the peak at 2940 cm⁻¹ is due to methylation of the amino groups of chitosan when they form part of the Schiff base for the covalent attachment with the protein. In the infrared spectrum of the microspheres carrying the antigen bound, the peak at 1516.7 cm⁻¹ disappears, characteristic of the free amino groups of the chitosan, while the peak at 1630 cm⁻¹, is diverted due to the occurrence of amide bonds between the glutaraldehyde and chitosan and protein.

In all spectra a peak at 1180 cm⁻¹ appears, characteristic of the Gantrez®AN119 aldehyde links.

### Example 2. Optimization of the concentration of antigen and microspheres that generate greater immune response

To initially optimize the concentration of antigen and microspheres needed that generate greater immune response, mice were vaccinated with different amounts of membrane antigen of *P. dicentrarchi* covalently coupled to the surface of the microspheres. In this case, mice were vaccinated on day 0 and were boosted at day 30 with 1, 2, 3 and 5 mg of particles carrying on their surface covalently bound antigen, and were used as controls mice vaccinated with antigen emulsified in an oily adjuvant (Freund's adjuvant, FCA), and mice immunized without adjuvant, only with particles (blank). As seen in Figure 4, mice immunized exclusively with the microspheres did not generate response anti-P. *dicentrarchi,* while mice that were immunized with antigen containing microspheres, produce a dose dependent antibody response, said response being maximal at the highest dose of microspheres carrying antigen. It can also be seen that, in this case, the vaccination of the mice with the antigen emulsified in FCA, generates the maximum response. Although FCA has been used for over 50 years for the production of antisera in animals (Freund J. 1956, Advances in Tuberculosis Research 7:130-48), at present, due to the seriousness and toxicity derived from their use, they are restricted to experimental studies and cannot be used in medical or veterinary clinic.

### Final formulation with microspheres for administration

Final formula or composition comprising the microspheres before being administered, that is, the final composition of the formulation for each dose (5 mg of microspheres are administered in suspension to each fish) is:
i. Chitosan: 0.472% (w/v).
ii. Gantrez®AN119: 0.452% (w/v).
iii. Glutaraldehyde: 0.037% (w/v).
iv. Antigen: 0.045% (w/v).
v. Milli Q ultrapure water, saline solution or dispersion medium: a.n.f. (amount needed for) 500 µl.

The w/v percentage is understood as the ratio between the weight of each component and the volume where the microspheres to be administered are suspended, multiplied by 100.

### Example 3. Verification of the immunogenicity in turbot of antigen-containing microspheres

To check the immunogenicity of microspheres in turbot carrying membrane antigen on their surface, fish were immunized according to the protocol outlined in Figure 1, with 5 mg of microspheres containing 230 µg of membrane antigen of *P. dicentrarchi* and with 230 µg of antigen without adjuvant. Fish serum obtained before immunization was used as control. Weekly, antibody levels in serum were measured by ELISA essays and the results obtained are presented in Figure 5. As can be seen, the fish vaccinated with microspheres + antigen showed kinetics with significant increase in antibody levels after primary and secondary immunization. By contrast, in fish immunized only with antigens, antibody levels were discreet, yielding only small increments after the immunizations. These results indicate that the microspheres act as adjuvants for the immune response, significantly increasing the levels of antibodies when the antigen is administered in conjunction with microspheres.

### Example 4. Comparison of vaccines with microspheres versus a vaccine with oily adjuvant

In this example the protective capacity of the vaccines developed in the present invention is illustrated in comparison with a vaccine using an oily adjuvant: in this case, Montanide ISA 763 A (Seppic, Paris, France) was used. Montanide ISA adjuvants are a group of surfactant/oil solutions based on the combination of surfactants with metabolizable non-mineral oils. They are prepared for use as an emulsion with the aqueous solution of the antigen. It seems that the effect of these adjuvants in antibody production is similar to Freund's adjuvant, however, the irritant response is usually much lower, and are approved by the Food and Drug Administration (FDA, USA) for veterinary use, demonstrating also there safety in fish vaccines.

In this experiment six groups of fish (each group contained 20 fish) were used which were individually vaccinated following the strategy shown in Figure 1: a) 230 µg of membrane antigen of *P. dicentrarchi* covalently coupled to the surface of the microspheres (5 mg microspheres/fish), b) 230 µg of antigen emulsified 1:1 with oily adjuvant Montanide ISA 763A, c) only 230 µg of antigen, d) with microspheres (5 mg/fish), d) with Montanide adjuvant, e) with physiological buffer (PBS). In this experiment it was determined: a) the level of protection obtained by the various vaccine formulations, b) the level of antibodies induced by the same, c) the degree of influence of vaccination on the growth of the fish immunized with the two types adjuvant.
a) Level of protection: With respect to mortality obtained after experimental infection with *P. dicentrarchi,* Figure 6 shows that the fish which presented a higher protection were vaccinated with microspheres superficially coupled with membrane antigen of the parasite, followed by fish vaccinated with the vaccine containing the antigen emulsified with oily adjuvant Montanide ISA 763A. Fish immunized only with microsphere, only with parasite antigen, or with PBS showed no protection, but immunized fish with only oily adjuvant Montanide showed some inspecific protection. This protection could be explained by high induction of inflammation produced by oily adjuvants generated in the peritoneal cavity of the fish. Moreover, the inspecific lack of induction of protection from microspheres could indicate that they do not induce peritoneal inflammation. The latter was confirmed after necropsy of fish, confirming the lack of any visible injury, irritation or adhesions in the peritoneum in fish vaccinated with the microspheres.
b) Level of antibodies: In this experiment the production of antibodies by different vaccines as indicated in Figure 6 was evaluated simultaneously. As seen in Figure 7, the vaccines containing antigen and the two adjuvants (Montanide adjuvant and microspheres), induced high levels of antibodies even in the first week of postimmunization, but there were no significant differences between them. On the contrary, after the second immunization, the antibody levels induced in fish following immunization with the microspheres and antigen were superior to those obtained following immunization with antigen emulsified in oily adjuvant Montanide. Thus, there seems to be a clear correlation between the levels of antibodies generated by the vaccine and the degree of protection obtained. Regarding other immunizations used in the experiment, it can be seen that the immunization with the adjuvant alone does not generate specific anti-*P*. *dicentrarchi* and immunization with antigen generates only a low antibody response.
c) Effects on growth: Finally the influence of vaccination with adjuvants on fish growth was analyzed. In this case, all the fish belonging to the group of turbot vaccinated with microspheres having covalently attached antigen, and of turbot immunized with the antigen emulsified in adjuvant Montanide were weighed. As seen in Figure 8, there is a significant difference between the weight of fish vaccinated with both adjuvants, being those vaccinated with microspheres the ones which showed higher growth, while fish vaccinated with the oily adjuvant presented lower growth.

## Claims

1. Microspheres comprising (i) a first biodegradable polymer of chitosan or a derivative thereof, (ii) a second biodegradable copolymer of methyl vinyl ether and maleic anhydride, or a derivative thereof, (iii) a crosslinking agent and (iv) one or several antigens.

2. Microspheres according to claim 1 wherein the antigen is covalently bonded to the outer surface of the microsphere.

3. Microspheres according to any of claims 1 to 2, which are prepared by adding antigen in a proportion of between 0.05 and 0.15% w/v, where w/v is the ratio between the amount of antigen in weight and the total volume of the suspension wherein the microspheres are suspended and the antigen is dissolved.

4. Microspheres according to any of claims 1 to 2, wherein the amount of antigen bound to the microsphere is between 2 and 7% by weight relative to the weight of the microsphere.

5. Microspheres according to any of claims 1 to 4 wherein the antigen is a component of a parasite that affects fish.

6. Microspheres according to claim 5, wherein the antigen is a component of at least one of the scuticociliated parasites *Philasterides dicentrarchi* or *Miamiensis avidus.*

7. Microspheres according to claim 6, wherein the antigen is a component of the membrane of at least one of the scuticociliated parasites *Philasterides dicentrarchi* or *Miamiensis avidus.*

8. Microspheres according to any of claims 1 to 7 wherein the crosslinking agent is selected from the group comprising: glutaraldehyde, formaldehyde, sodium tripolyphosphate, genipin, glyoxal and ethylene glycol diglycidyl ether.

9. Microspheres according to any of claims 1 to 8, which are prepared by adding a crosslinking agent in a proportion of between 0.01 and 0.1 % w/v, where w/v is the ratio between the weight of crosslinking agent and the total volume of the dispersion of the two polymers that form part of the microsphere.

10. Microspheres according to any of claims 1 to 9 which are prepared by adding the first polymer in a proportion of between 0.12 and 0.5% (w/v), and the copolymer in a proportion of between 0.15 and 0,5% (w/v), w/v meaning the ratio between the weight of polymer or copolymer and the total final volume of the dispersion of the two polymers that form part of the microsphere.

11. Microspheres according to any of claims 1 to 10 wherein the diameter of the microsphere is between 1 and 10 µm.

12. Microspheres according to any of claims 1 to 11 comprising:
i. Chitosan polymer: 25-75% (w/w)
ii. Copolymer of methyl vinyl ether-maleic anhydride: 25-75% (w/w)
iii. Crosslinking agent: 2.4-5% (w/w)
iv. Antigen: 2-7% (w/w),
where w/w is the ratio between the weight of each component and the total weight of the microsphere.

13. Microspheres according to any of claims 1 to 12 wherein said microspheres are lyophilized.

14. A pharmaceutical composition comprising the microspheres according to any of claims 1 to 13 together with a dispersion medium.

15. Pharmaceutical composition according to preceding claim wherein the dispersion medium comprises: carboxymethylcellulose, polysorbate, lecithin, paraffin wax or a natural oil.

16. Pharmaceutical composition according to any of claims 14 to 15 wherein the microspheres are in a proportion between 0.1-2% w/v.

17. Pharmaceutical composition according to any of claims 14 to 15 comprising:
i. Chitosan polymer: 0.25-0.75% (w/v)
ii. Copolymer of methyl vinyl ether-maleic anhydride: 0.25-0.75%. (w/v)
iii. Crosslinking agent: 0.024 to 0.05% (w/v)
iv. Antigen: 0.02-0.07% (w/v)
v. dispersion medium

18. Use of the pharmaceutical composition according to any of claims 14 to 17 for the manufacture of a medicament for stimulating and/or inducing an immune response in a fish.

19. Use of the pharmaceutical composition according to any of claims 14 to 17 for the preparation of a vaccine for a fish.

20. Use of the pharmaceutical composition according to the preceding claim wherein the fish is selected from the group comprising flatfish and bass.

21. Use of the pharmaceutical composition according to the preceding claim wherein the fish is flounder or turbot.

22. Use of the pharmaceutical composition according to any of claims 18 to 21, wherein the composition is in a form suitable for parenteral administration.

23. Use of the pharmaceutical composition according to any of claims 18 to 22, wherein the pharmaceutical composition is administered in a dose of between 0.1 and 0.5 g per kilogram of the subject's weight.

24. Method for preparing the microspheres according to any of claims 1 to 13 comprising the steps of:
i. Preparing a dispersion of a copolymer of methyl vinyl ether and maleic anhydride in an organic solvent
ii. Preparing a dispersion of a chitosan polymer in an aqueous solvent
iii. Incorporating the dispersion of step i) with the dispersion of step ii)
iv. Addition of a crosslinking agent.
v. Obtaining microspheres by one of the procedures selected from the group comprising: atomization, emulsion/solvent evaporation and coacervation.
vi. Antigen binding to the outer surface of the microsphere.

25. Method for preparing the microspheres according to claim 24, wherein in step v) microspheres are obtained by atomization.

26. Method for preparing the microspheres according to claim 25 wherein the atomization process takes place under the following conditions: atomization temperature: 72°C, feed rate: 3-4 ml/min and air pressure 500l/h.

27. Method for preparing the microspheres according to any of claims 24 to 26, wherein in step i), the organic solvent is acetone, and methyl vinyl ether copolymer is incorporated at an initial concentration of between 0.15 and 0.5 % w/v in relation to the total volume of the dispersion of the two polymers.

28. Method for preparing the microspheres according to any of claims 24 to 27, wherein in step ii), the solvent is an acetic acid solution and the chitosan polymer is employed as chitosan hydrochloride at an initial concentration of between 0.12 and 0.5% w/v in relation to the total volume of the dispersion of the two polymers.

29. Method for preparing the microspheres according to any of claims 24 to 28, wherein in step iii), the mixture is carried out under magnetic stirring.

30. Method for preparing the microspheres according to any of claims 24 to 29, wherein in step iv), the crosslinking agent is glutaraldehyde in a proportion of between 0.01 and 0.1% w/v in relation to the total volume of the dispersion of the two polymers.

31. Method for preparing the microspheres according to any of claims 24 to 30, wherein in step vi), the binding of the antigen to the outer surface of the microsphere is of covalent type.

32. Method for preparing the microspheres according to claim 31, wherein the binding of antigen is performed by incorporating an antigen solution over a suspension of microspheres obtained in step v), and wherein said antigen solution is in a proportion of between 0.05 and 0.15% w/v with respect to the total volume of the suspension wherein the microspheres are suspended and the antigen is dissolved.

33. Method for preparing the microspheres according to claim 32, wherein the antigen binding is performed by incorporating an antigen solution of 0.1% w/v over a suspension of microspheres obtained in step v), maintaining agitation and centrifugating to isolate the microspheres.
